# EUROPÄISCHE PATENTSCHRIFT

(11) **EP 1 676 593 B1**
(45) Veröffentlichungstag und Bekanntmachung des Hinweises auf die Patenterteilung: **08.08.2007**
(21) Anmeldenummer: 05023826.0
(22) Anmeldetag: 02.11.2005
(51) Int. Cl.: A61M 16/20

(54) **Vorrichtung zur Beatmung sowie Verfahren zur Steuerung eines Beatmungsgerätes**
Breathing device and method of controlling a breathing device
Appareil respiratoire et méthode de commande d'un appareil respiratoire

(30) Priorität: 29.12.2004 DE 102004063158
(43) Veröffentlichungstag der Anmeldung: 05.07.2006
(73) Patentinhaber: Weinmann Geräte für Medizin GmbH & Co. KG, 22525 Hamburg (DE)
(72) Erfinder: Paesch, Rainer, 25451 Quickborn (DE); Dietz, Florian, 23554 Lübeck (DE)
(74) Vertreter: Klickow, Hans-Henning

(56) Entgegenhaltungen:
- EP-A- 0 671 181
- EP-A- 1 059 096
- US-A1- 2004 211 422

## Beschreibung

Die Erfindung betrifft eine Vorrichtung zur Beatmung, die eine Druckgasversorgung sowie eine Steuerung für die Druckgasversorgung aufweist.

Die Erfindung betrifft darüber hinaus ein Verfahren zur Steuerung eines Beatmungsgerätes, bei dem ein erzeugter Druck unter Verwendung eines im Bereich eines einem Patienteninterface zugewandten Endes eines Beatmungsschlauches positionierten ventils gesteuert wird, sowie bei dem ein von einer Druckgasversorgung bereitgestellter Druck unter Verwendung mindestens eines meßtechnisch erfaßten Signals geregelt wird.

Derartige Vorrichtungen zur Beatmung können beispielsweise zur Durchführung einer PEEP-Beatmung (positiver Endexpiratorischer Druck) oder einer EPAP-Beatmung (expiratorischer positiver Atemwegsdruck) ausgebildet sein. Bekannt sind derartige Beatmungsgeräte mit Regeleinrichtungen, bei denen ein magnetisches Druckentlastungsventil eingesetzt wird. Nachteilig bei den bekannten Verfahren und Vorrichtungen sind das Auftreten unstetiger Druckentlastungen sowie vergleichsweise große Toträume, die bei Druckerhöhungen und Druckabsenkungen zu zeitlichen Verzögerungen beim Druckaufbau bzw. bei der Druckabsenkung führen.

Aus der US 2004/211422A1 ist bereits eine Vorrichtung zur Beatmung bekannt, die eine Druckgasversorgung mit einem Beatmungsschlauch aufweist. Im Bereich eines geräteseitigen Endes des Beatmungsschlauches ist ein steuerbares Ventil angeordnet. Die Druckgasversorgung ist über eine Ausströmöffnung mit einer Umgebung verbindbar. Eine Druckregelung erfolgt unter Verwendung eines Druckreglers und eines Drucksensors .

In der EP-A-0 671 181 wird ebenfalls eine Vorrichtung zur Beatmung beschrieben, die einen an eine Druckgasversorgung angeschlossenen Beatmungsschlauch aufweist. Eine Druckregelung erfolgt unter Verwendung eines Druckreglers und eines Drucksensors, wobei ein konkret anliegender Druck unter Verwendung eines steuerbaren Ventils eingestellt wird.

Aufgabe der vorliegenden Erfindung ist es, eine Vorrichtung der einleitend genannten Art derart zu konstruieren, daß bei einfachem konstruktiven Aufbau eine kontinuierliche und schnelle Regelung ermöglicht wird.

Diese Aufgabe wird erfindungsgemäß dadurch gelöst, daß im Bereich eines einem Patienteninterface zugewandten Endes eines an die Druckgasversorgung angeschlossenen Beatmungsschlauches ein mit der Steuerung verbundenes steuerbares Ventil angeordnet ist, daß die Druckgasversorgung über eine Blende wenigstens zeitweise mit einer Umgebung verbunden ist und daß die Blende getrennt vom Ventil ausgebildet ist, daß ein als Teil der Steuerung ausgebildeter Druckregler an einen Drucksensor zur Druckerfassung angeschlossen ist und daß im Bereich eines Anschlusses des Beatmungsschlauches an die Verbindungskammer ein Rückschlagventil angeordnet ist.

Weitere Aufgabe der vorliegenden Erfindung ist es, ein Verfahren der einleitend genannten Art derart zu verbessern, daß ein verbessertes Regelverhalten hinsichtlich des bereitgestellten Druckes unterstützt wird.

Diese Aufgabe wird erfindungsgemäß dadurch gelöst, daß mindestens zeitweilig eine Leckage gegenüber einer Umgebung durch eine Druckluftableitung über eine Blende erzeugt wird und daß eine Atemgasrückströmung durch ein Rückschlagventil im Bereich des Beatmungsschlauches abgesperrt wird.

Durch die Verwendung der Blende ist es möglich, den expiratorischen Druck in einem weiten Druckbereich stetig regel bar zu machen. Über die Blende wird ein kontinuierlicher Gasfluß gegenüber einer Umgebung erzeugt und in Abhängigkeit vom jeweiligen Gasfluß fällt ein definierter Druck an der Blende ab. Dieser Druck kann meßtechnisch erfaßt und in einer Regeleinrichtung mit einem vorgegebenen Sollwert verglichen werden. Durch eine entsprechende Regelcharakteristik kann der Abgabedruck eines Patientenventils eingestellt werden.

Als Beatmungszugang für einen Patienten kann beispielsweise eine Maske, ein Tubus, ein Helm oder ein Tracheostoma verwendet werden.

Unter Verwendung der erfindungsgemäßen Vorrichtung und des erfindungsgemäßen Verfahrens lassen sich Zeitkonstanten zur Druckentlastung realisieren, die in einem Bereich von 10 bis 50 Millisekunden liegen.

Die entstehenden Leckageverluste können dadurch reduziert werden, daß die Blende mit einem Schaltventil in Reihe geschaltet ist.

Zur Unterstützung eines Saugbetriebes ist vorgesehen, daß parallel zur Reihenschaltung der Blende und des Schaltventils ein Rückschlagventil angeordnet ist.

Die Vermeidung inspiratorischer Leckagen wird dadurch unterstützt, daß die Blende an eine Venturidüse angeschlossen ist.

Ein einfacher konstruktiver Aufbau wird dadurch unterstützt, daß der Drucksensor im Bereich einer Verbindungskammer angeordnet ist, die zwischen einer Druckgasquelle und dem Beatmungsschlauch positioniert ist.

Ein schnelles Systemverhalten durch Vermeidung einer Entleerung des Beatmungsschlauches bei einer Druckabsenkung wird dadurch erreicht, daß im Bereich eines Anschlusses des Beatmungsschlauches an die Verbindungskammer ein Rückschlagventil angeordnet ist.

Eine pneumatische Steuerung des Patientenventils wird dadurch unterstützt, daß die Verbindungskammer über einen Steuerschlauch an das Patientenventil angeschlossen ist.

Eine weitere Ausführungsvariante besteht darin, daß sowohl die Blende als auch das parallel zur Blende angeordnete Rückschlagventil an die Verbindungskammer angeschlossen sind.

Darüber hinaus ist daran gedacht, daß das Rückschlagventil die Venturidüse mit der Umgebung verbindet.

Verschmutzungen der Blende werden auch ohne Verwendung eines Rückschlagventils dadurch vermieden, daß die Blende an einen Verbindungsbereich zwischen der Venturidüse und dem Beatmungsschlauch angeschlossen ist.

Ein Saugbetrieb wird auch dadurch unterstützt, daß das Rückschlagventil parallel zur Blende angeordnet ist.

Ein Eindringen von Verunreinigungen in den Bereich der Blende bei der Durchführung eines Saugbetriebes kann dadurch verhindert werden, daß die Blende in Reihe mit einem Rückschlagventil angeordnet ist.

Eine weitere Ausführungsvariante besteht darin, daß die Blende an die Verbindungskammer und das Rückschlagventil an die Venturidüse angeschlossen ist.

Eine vereinfachte Ausführungsform wird dadurch bereitgestellt, daß die Blende und das Schaltventil an die Verbindungskammer angeschlossen sind und daß die Verbindungskammer über ein Proportionalventil direkt mit der Druckgasquelle verbunden ist.

Eine nochmals vereinfachte konstruktive Realisierung wird dadurch unterstützt, daß die Verbindungskammer direkt an die Druckgasquelle angeschlossen ist.

Ein typisches Anwendungsgebiet besteht darin, daß eine Ausbildung zur Durchführung von Heimbeatmungen vorliegt.

Eine weitere Anwendung wird dadurch definiert, daß eine Ausbildung zur Durchführung von Notfallbeatmungen vorliegt.

Ebenfalls ist daran gedacht, daß eine Ausbildung zur Durchführung'von Transportbeatmungen vorliegt. Eine weitere Variante besteht in einer Anwendung bei Intentivbeatmungen.

Gemäß einer typischen Ausführungsform ist vorgesehen, daß ein exspiratorischer Druck geregelt wird.

Eine genaue Regelung wird dadurch unterstützt, daß die Druckregelung unter Verwendung eines Proportionalventils durchgeführt wird.

Eine besonders gute Regelungsqualität kann dadurch erreicht werden, daß eine zeitlich kontinuierliche oder quasi kontinuierliche Regelung durchgeführt wird.

In den Zeichnungen sind Ausführungsbeispiele der Erfindung schematisch dargestellt. Es zeigen:
- Fig. 1: eine perspektivische Darstellung eines Beatmungsgerätes mit verbindungsschlauch einer Beatmungsmaske,
- Fig. 2: ein Blockschaltbild zur Veranschaulichung der wesentlichen Funktionskomponenten bei einer maximalen Ausführung,
- Fig. 3: eine gegenüber der Darstellung in Fig. 2 abgewandelte Ausführungsform,
- Fig. 4: eine Anordnung ohne Verwendung eines 3/2-Wege- Ventils,
- Fig. 5: eine gegenüber der Ausführungsform in Fig. 4 abgewandelte Ausführungsform bei Verwendung eines zusätzlichen Rückschlagventils,
- Fig. 6: eine weitere Ausführungsform ohne 3/2-Wege-Ventil,
- Fig. 7: eine Ausführungsform ohne Venturi-Düse und
- Fig. 8: eine Ausführungsform sowohl ohne Venturi-Düse als auch ohne 3/2-Wege-Ventil.

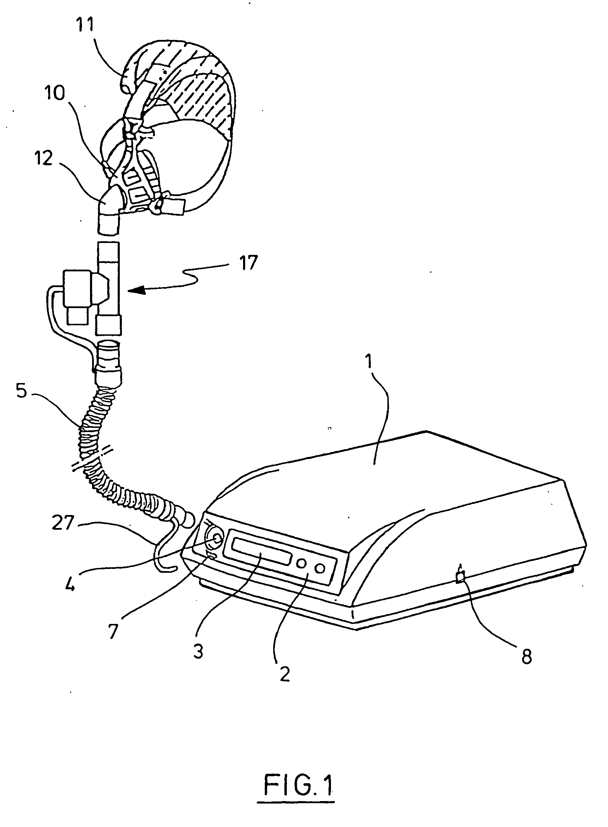

Fig. 1 zeigt den grundsätzlichen Aufbau einer Vorrichtung zur Beatmung. Im Bereich eines Gerätegehäuses (1) mit Bedienfeld (2) sowie Anzeige (3) ist in einem Geräteinnenraum eine Atemgaspumpe angeordnet. Über eine Kopplung (4) wird ein Verbindungsschlauch (5) angeschlossen. Entlang des Verbindungsschlauches (5) kann ein zusätzlicher Steuerschlauch (27) verlaufen, der über einen Druckeingangsstutzen (7) mit dem Gerätegehäuse (1) verbindbar ist. Zur Ermöglichung einer Datenübertragung weist das Gerätegehäuse (1) eine Schnittstelle (8) auf.

Im Bereich einer dem Gerätegehäuse (1) abgewandten Ausdehnung des Verbindungsschlauches (5) ist als Ausatmungselement ein Patientenventil (17) angeordnet.

Fig. 1 zeigt darüber hinaus eine Beatmungsmaske (10), die als Nasalmaske ausgebildet ist. Eine Fixierung im Bereich eines Kopfes eines Patienten kann über eine Kopfhaube (11) erfolgen. Im Bereich ihrer dem Verbindungsschlauch (5) zugewandten Ausdehnung weist die Beatmungsmaske (10) ein Kupplungselement (12) auf.

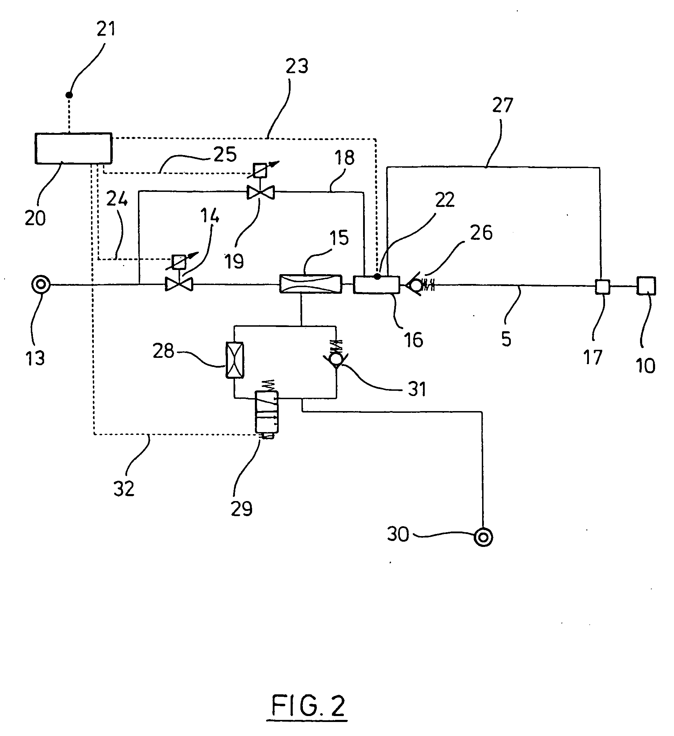

Fig. 2 zeigt in einem Blockschaltbild die wesentlichen zwischen einer Druckgasquelle (13) und der Beatmungsmaske (10) angeordneten Funktionskomponenten zur Ausbildung der Druckgasversorgung. Die Druckgasquelle (13) ist über ein Proportionalventil (14) an eine Venturidüse (15) angeschlossen. Ein Ausgang der Venturidüse (15) mündet in eine Verbindungskammer (16) ein. Die Verbindungskammer (16) ist über den Verbindungsschlauch (5) an ein Patientenventil (17) angeschlossen. Das Patientenventil (17) ist in räumlicher Nähe zur Beatmungsmaske (10) positioniert.

Als Bypaß zur Venturidüse (15) verläuft zwischen der Druckgasquelle (13) und der Verbindungskammer (16) eine Bypaßleitung (18), in deren Bereich ein weiteres Proportionalventil (19) angeordnet ist. Die Proportionalventile (14, 19) sind jeweils an eine Steuerung (20) angeschlossen, der über an einen Sollwertanschluß (21) ein Sollwert für das PEEP-Druckniveau oder ein EPAP-Druckniveau zugeführt wird.

Alternativ zu mindestens einem der Proportionalventile (14, 19) ist es auch möglich, zur Drucksteuerung ein verstellbares Gebläse oder einen Hubkolben zu verwenden. Darüber hinaus ist auch an die Verwendung von elektromechanischen, pneumatischen, magnetischen oder elektromagnetischen Stellgliedern gedacht.

Im Bereich der Verbindungskammer (16) ist ein Drucksensor (22) angeordnet, der über eine Sensorleitung (23) mit der Steuerung (20) verbunden ist. Die Proportionalventile (14, 19) sind über Steuerleitungen (24, 25) mit der Steuerung (20) verbunden.

In einem Übergangsbereich des Verbindungsschlauches (5) zur Verbindungskammer (16) ist ein Rückschlagventil (26) angeordnet, das bei einer Druckabsenkung der Verbindungskammer (16) eine Entleerung des Verbindungsschlauches (5) in die Verbindungskammer (16) hinein vermeidet. Von der verbindungskammer (16) zum Patientenventil (17) verläuft ein Steuerschlauch (27).

Die Venturidüse (15) ist über die Reihenschaltung einer Blende (28) und eines Schaltventils (29) mit einer Umgebung (30) verbunden. Parallel zur Blende (28) ist ein Rückschlagventil (31) positioniert, das eine Druckluftweiterleitung von der Venturidüse (15) in Richtung auf die Umgebung (30) verhindert. Das Schaltventil (29) ist als 3/2-Wege-Ventil ausgebildet und über eine Steuerleitung (32) mit der Steuerung (20) verbunden.

Durch die Verwendung der Blende (28) und mindestens eines der Proportionalventile (14, 19) ist es möglich, den expiratorischen Druck in einem relativ großen Druckbereich stetig zu regeln. Über die Blende (28) und das Schaltventil (29) wird ein kontinuierlicher Gasfluß zur Umgebung (30) erzeugt. Der an der Blende (28) abfallende Druck stellt sich auch sowohl in der Verbindungskammer (16) als auch im Bereich des Steuerschlauches (27) ein. Durch dieses im wesentlichen gleiche Druckniveau ist es möglich, den resultierenden Druck im Bereich des Patientenventils (17) vorzugeben.

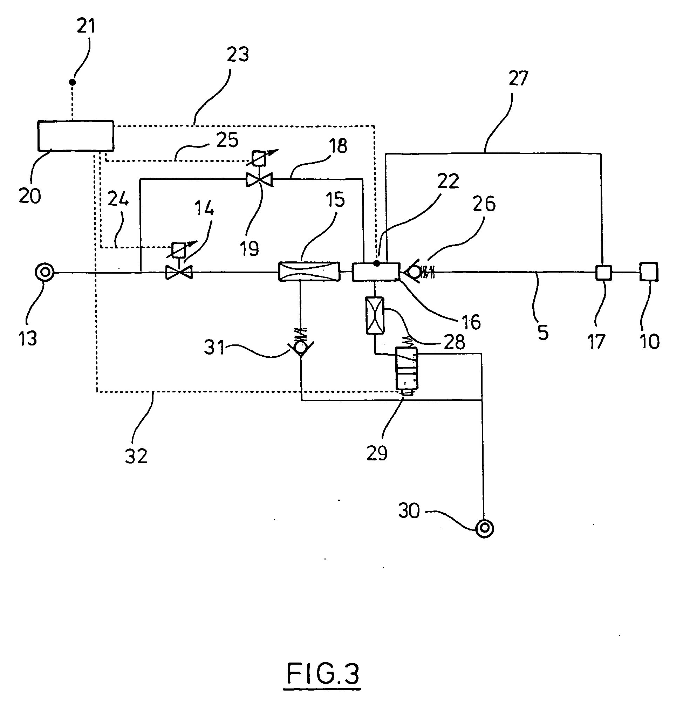

Bei der Ausführungsform gemäß Fig. 3 ist die Blende (28) direkt an die Verbindungskammer (16) angeschlossen. Das Rückschlagventil (31) ist bei dieser Ausführungsform an die Venturidüse (15) angeschlossen und verläuft parallel zur Reihenschaltung aus der Blende (28) und dem Schaltventil (29).

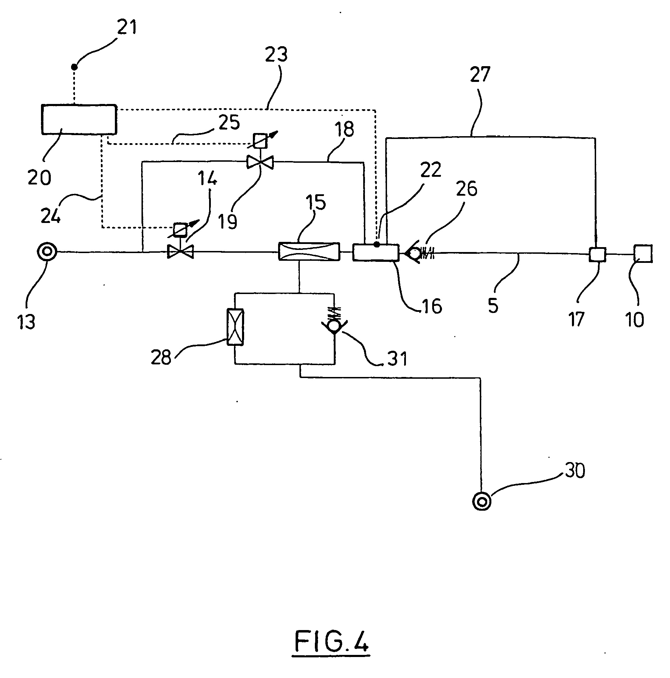

Fig. 4 zeigt eine Ausführungsform, bei der als Abwandlung zur Ausführungsform gemäß Fig. 2 die Blende (28) ohne Verwendung eines Schaltventils (29) direkt an die Umgebung (30) angeschlossen ist. Alternativ zu der einen in Fig. 4 eingezeichneten Venturidüse (15) können auch eine Mehrzahl von Venturidüsen (15) mit Bypaßleitungen (18) verwendet werden. Durch das Rückschlagventil (31) gemäß Fig. 2 bis Fig. 4 ist es möglich, daß die Venturidüse (15) eine Ansaugung von Umgebungsluft vornimmt und der Gasmischung zuführt. Gemäß einer typischen Ausführungsform ist das Proportionalventil (14) für einen geringeren Durchfluß als das Proportionalventil (19) ausgelegt. Eine Drucksteuerung unter Verwendung des Proportionalventils (14) weist somit den Vorteil einer feineren Einstellbarkeit auf.

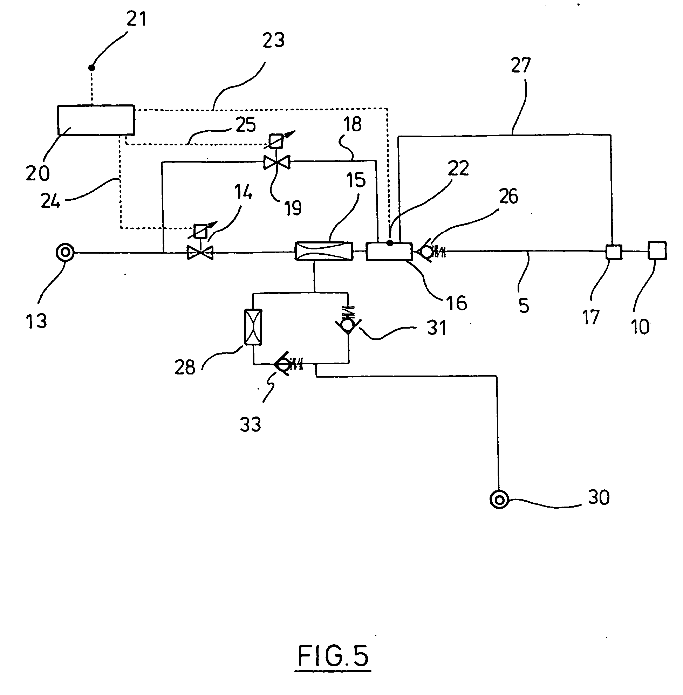

Gemäß der Ausführungsform in Fig. 5 ist als Abwandlung zu Fig. 4 zwischen der Blende (28) und der Umgebung (30) ein weiteres Rückschlagventil (33) angeordnet, das ein Eindringen von Fremdkörpern in den Bereich der Blende (28) bei einem Saugbetrieb der Venturidüse (15) verhindert. Generell ermöglicht die Verwendung einer Venturidüse (15) die Vermeidung eines Schaltventils (29) bei gleichzeitiger Minimierung der Verluste an Versorgungsgas bei der Durchführung eines Mischbetriebes. Bei einem derartigen Mischbetrieb wird Umgebungsluft angesaugt und es strömt über die Blende (28) kein Atemgas nach außen.

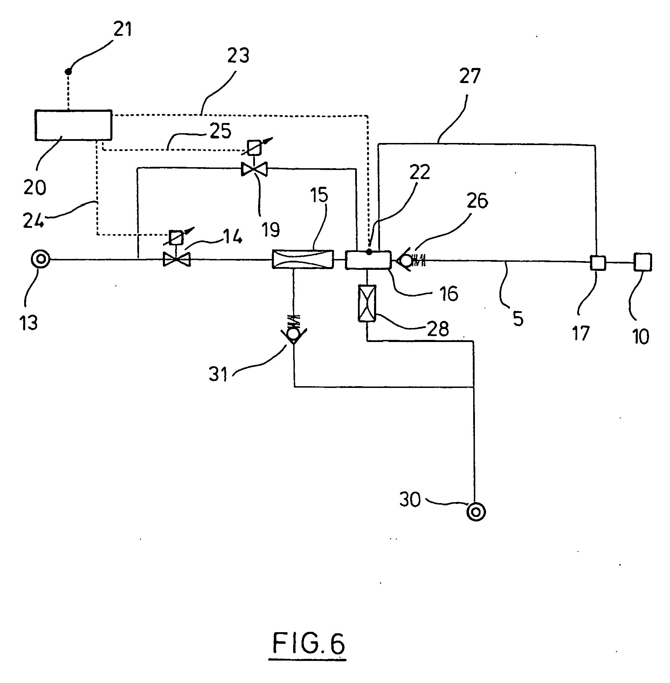

Gemäß der Ausführungsform in Fig. 6 ist als Abwandlung zu Fig. 4 lediglich das Rückschlagventil (31) direkt zwischen der Venturidüse (15) und der Umgebung (30) angeordnet, die Blende (28) verbindet hingegen direkt die Verbindungskammer (16) mit der Umgebung (30). Auch diese Ausführungsform ermöglicht somit eine Ansaugung von Umgebungsluft durch die Venturidüse (15) über das Rückschlagventil (31) bei gleichzeitigem kontinuierlichen Gasfluß über die Blende (28).

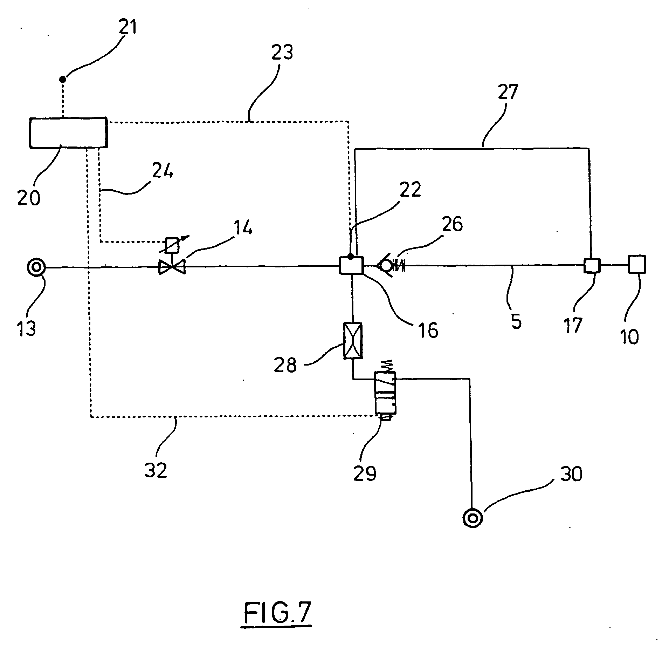

Gemäß der Ausführungsform in Fig. 7 wird die Druckgasversorgung (13) direkt über das Proportionalventil (14) an die Verbindungskammer (16) angeschlossen. Es erfolgt somit eine Konstruktion ohne Verwendung einer Venturidüse (15). Die Blende (28) ist an die Verbindungskammer (16) angeschlossen und über das Schaltventil (29) mit der Umgebung (30) verbindbar.

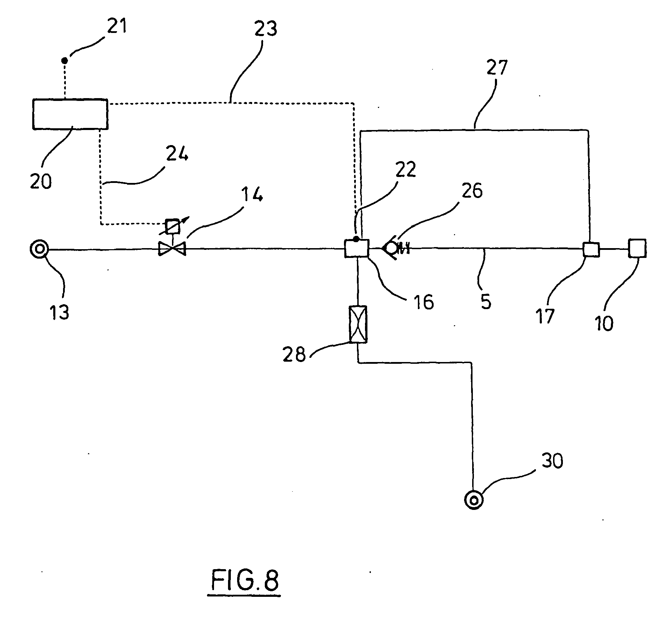

Gemäß der Ausführungsform in Fig. 8 erfolgt eine weitere Vereinfachung gegenüber der Ausführungsform in Fig. 7 durch ein weglassen des Schaltventils (29). Die Blende (28) verbindet hierdurch permanent die Verbindungskammer (16) mit der Umgebung (30).

## Patentansprüche

1. Vorrichtung zur Beatmung, die eine Druckgasversorgung (13) sowie eine Steuerung für die Druckgasversorgung (13) aufweist, wobei ein als Teil der Steuerung (20) ausgebildeter Druckregler an einen Drucksensor (22) zur Druckerfassung angeschlossen ist, **dadurch gekennzeichnet, daß** im Bereich eines einem Patienteninterface zugewandten Endes eines an die Druckgasversorgung (13) angeschlossenen Beatmungsschlauches (5) ein mit der Steuerung (20) verbundenes steuerbares Ventil (17) angeordnet ist, daß die Druckgasversorgung (13) über eine Blende (28) wenigstens zeitweise mit einer Umgebung verbunden ist und daß die Blende (28) getrennt vom Ventil (17) ausgebildet ist, und daß im Bereich eines Anschlusses des Beatmungsschlauches (5) an die Verbindungskammer (16) ein Rückschlagventil (26) angeordnet ist.

2. Vorrichtung nach Anspruch 1, **dadurch gekennzeichnet, daß** die Blende (28) mit einem Schaltventil (29) in Reihe geschaltet ist.

3. Vorrichtung nach Anspruch 1 oder 2, **dadurch gekennzeichnet, daß** parallel zur Reihenschaltung der Blende (28) und des Schaltventils (29) ein Rückschlagventil (31) angeordnet ist.

4. Vorrichtung nach einem der Ansprüche 1 bis 3, **dadurch gekennzeichnet, daß** die Blende (28) an eine Venturidüse (15) angeschlossen ist.

5. Vorrichtung nach einem der Ansprüche 1 bis 4, **dadurch gekennzeichnet, daß** der Drucksensor (22) im Bereich einer Verbindungskammer (16) angeordnet ist, die zwischen einer Druckgasquelle (13) und dem Beatmungsschlauch (5) positioniert ist.

6. Vorrichtung nach einem der Ansprüche 1 bis 5, **dadurch gekennzeichnet, daß** die Verbindungskammer (16) über einen Steuerschlauch (27) an das Patientenventil (17) angeschlossen ist.

7. Vorrichtung nach einem der Ansprüche 1 bis 6, **dadurch gekennzeichnet, daß** sowohl die Blende (28) als auch das parallel zur Blende (28) angeordnete Rückschlagventil (31) an die Verbindungskammer (16) angeschlossen sind.

8. Vorrichtung nach einem der Ansprüche 1 bis 7, **dadurch gekennzeichnet, daß** das Rückschlagventil (31) die Venturidüse (15) mit der Umgebung verbindet.

9. Vorrichtung nach einem der Ansprüche 4 und 5 bis 8 wenn abhängig von 4, **dadurch gekennzeichnet, daß** die Blende (28) an einen Verbindungsbereich zwischen der Venturidüse (15) und dem Beatmungsschlauch (5) angeschlossen ist.

10. Vorrichtung nach einem der Ansprüche 1 bis 9, **dadurch gekennzeichnet, daß** das Rückschlagventil (31) parallel zur Blende (28) angeordnet ist.

11. Vorrichtung nach einem der Ansprüche 1 bis 10, **dadurch gekennzeichnet, daß** die Blende (28) in Reihe mit einem Rückschlagventil (33) angeordnet ist .

12. Vorrichtung nach einem der Ansprüche 4 und 5 bis 11 wenn abhängig von 4, **dadurch gekennzeichnet, daß** die Blende (28) an die Verbindungskammer (16) und das Rückschlagventil (31) an die Venturidüse (15) angeschlossen ist.

13. Vorrichtung nach einem der Ansprüche 1 bis 11, **dadurch gekennzeichnet, daß** die Blende (28) und das Schaltventil (29) an die Verbindungskammer (16) angeschlossen sind und daß die Verbindungskammer (16) über ein Proportionalventil (14) direkt mit der Druckgasquelle (13) verbunden ist.

14. Vorrichtung nach einem der Ansprüche 1 bis 11, **dadurch gekennzeichnet, daß** die Verbindungskammer (16) direkt an die Druckgasquelle (13) angeschlossen ist.

15. Vorrichtung nach einem der Ansprüche 1 bis 14, **dadurch gekennzeichnet, daß** eine Ausbildung zur Durchführung von Heimbeatmungen vorliegt.

16. Vorrichtung nach einem der Ansprüche 1 bis 14, **dadurch gekennzeichnet, daß** eine Ausbildung zur Durchführung von Notfallbeatmungen vorliegt.

17. Vorrichtung nach einem der Ansprüche 1 bis 14, **dadurch gekennzeichnet, daß** eine Ausbildung zur Durchführung von Transportbeatmungen vorliegt.

18. Vorrichtung nach einem der Ansprüche 1 bis 14, **dadurch gekennzeichnet, daß** eine Ausbildung zur Durchführung von Intensivbeatmungen vorliegt.

19. Verfahren zur Steuerung eines Beatmungsgerätes, bei dem ein von einer Druckgasversorgung (13) bereitgestellter Druck unter Verwendung mindestens eines meßtechnisch erfaßten Signals geregelt wird, **dadurch gekennzeichnet, daß** ein erzeugter Druck unter Verwendung eines im Bereich eines einem Patienteninterface zugewandten Endes eines Beatmungsschlauches (5) positionierten Ventils gesteuert wird, sowie mindestens zeitweilig eine Leckage gegenüber einer Umgebung durch eine Druckluftableitung über eine von dem Ventil getrennt ausgebildete Blende (28) erzeugt wird und daß eine Atemgasrückströmung durch ein Rückschlagventil (26) im Bereich des Beatmungsschlauches (5) abgesperrt wird.

20. Verfahren nach Anspruch 19, **dadurch gekennzeichnet, daß** ein exspiratorischer Druck geregelt wird.

21. Verfahren nach Anspruch 19 oder 20, **dadurch gekennzeichnet, daß** die Druckregelung unter Verwendung eines Proportionalventils (19) durchgeführt wird.

22. Verfahren nach einem der Ansprüche 19 bis 21, **dadurch gekennzeichnet, daß** eine zeitlich kontinuierliche Regelung durchgeführt wird.

23. Verfahren nach einem der Ansprüche 19 bis 21, **dadurch gekennzeichnet, daß** eine zeitlich quasi kontinuierliche Regelung durchgeführt wird.

## Claims

1. Respirator device, comprising a compressed gas supply (13) and a control system for the compressed gas supply (13), and a pressure regulator constituting part of the control system (20) is connected to a pressure sensor (22) for detecting the pressure, **characterised in that** a controllable valve (17) connected to the control system (20) is disposed in the region of one end of a respiratory tube (5) facing the patient interface, connected to the compressed gas supply (13), and the compressed gas supply (13) is at least intermittently connected to an ambient environment via a diaphragm (28), and the diaphragm (28) is disposed separately from the valve (17), and a non-return valve (26) is disposed in the region of a connection of the respiratory tube (5) to the connecting chamber (16).

2. Device as claimed in claim 1, **characterised in that** the diaphragm (28) is connected in series to a switch valve (29).

3. Device as claimed in claim 1 or 2, **characterised in that** a non-return valve (31) is disposed parallel with the serial connection of the diaphragm (28) and the switch valve (29).

4. Device as claimed in one of claims 1 to 3, **characterised in that** the diaphragm (28) is connected to a Venturi nozzle (15).

5. Device as claimed in one of claims 1 to 4, **characterised in that** the pressure sensor (22) is disposed in the region of a connecting chamber (16), which is positioned between a compressed gas source (13) and the respiratory tube (5).

6. Device as claimed in one of claims 1 to 5, **characterised in that** the connecting chamber (16) is connected to the patient valve (17) via a control tube (27) .

7. Device as claimed in one of claims 1 to 6, **characterised in that** both the diaphragm (28) and the non-return valve (31) disposed parallel with the diaphragm (28) are connected to the connecting chamber (16).

8. Device as claimed in one of claims 1 to 7, **characterised in that** the non-return valve (31) connects the Venturi nozzle (15) to the ambient environment.

9. Device as claimed in one of claims 4 and 5 to 8 if dependent on claim 4, **characterised in that** the diaphragm (28) is connected to a connecting region between the Venturi nozzle (15) and the respiratory tube (5).

10. Device as claimed in one of claims 1 to 9, **characterised in that** the non-return valve (31) is disposed parallel with the diaphragm (28).

11. Device as claimed in one of claims 1 to 10, **characterised in that** the diaphragm (28) is disposed in series with a non-return valve (33).

12. Device as claimed one of claims 4 and 5 to 11 if dependent on claim 4, **characterised in that** the diaphragm (28) is connected to the connecting chamber (16) and the non-return valve (31) is connected to the Venturi nozzle (15).

13. Device as claimed in one of claims 1 to 11, **characterised in that** the diaphragm (28) and the switch valve (29) are connected to the connecting chamber (16) and the connecting chamber (16) is connected directly to the compressed gas source (13) via a proportional valve (14).

14. Device as claimed in one of claims 1 to 11, **characterised in that** the connecting chamber (16) is connected directly to the compressed gas source (13).

15. Device as claimed in one of claims 1 to 14, **characterised in that** it has a mode in which it can be used for respiratory applications in the home.

16. Device as claimed in one of claims 1 to 14, **characterised in that** it has a mode in which it can be used for emergency respiratory applications.

17. Device as claimed in one of claims 1 to 14, **characterised in that** it has a mode in which it can be used for respiratory applications during transportation.

18. Device as claimed in one of claims 1 to 14, **characterised in that** it has a mode in which it can be used for intensive care respiratory applications.

19. Method of controlling a respirator device, whereby a pressure delivered by a compressed gas supply (13) can be regulated using at least one signal detected by a measuring system, **characterised in that** a generated pressure is controlled using a valve positioned in the region of an end of a respiratory tube (5) facing a patient interface, and a leakage to the ambient environment is at least intermittently generated by diverting compressed air via a diaphragm (28) which is separate from the valve, and a respiratory gas return flow is blocked by a non-return valve (26) in the region of the respiratory tube (5).

20. Method as claimed in claim 19, **characterised in that** an expiratory pressure is regulated.

21. Method as claimed in claim 19 or 20, **characterised in that** the pressure regulation is operated using a proportional valve (19).

22. Method as claimed in one of claims 19 to 21, **characterised in that** a regulation is operated continuously in terms of time.

23. Method as claimed in one of claims 19 to 21, **characterised in that** a regulation is operated almost continuously in terms of time.

## Revendications

1. Dispositif respiratoire qui présente une alimentation (13) en gaz sous pression ainsi qu'une commande de l'alimentation (13) en gaz sous pression, un régulateur de pression qui constitue une partie de la commande (20) étant raccordé à une sonde de pression (22) qui détecte la pression, **caractérisé en ce qu'**une soupape (17) asservie reliée à la commande (20) est disposée dans la zone d'une extrémité, tournée vers une interface de patient, d'un tuyau respiratoire flexible (5) raccordé à l'alimentation (13) en gaz sous pression, **en ce qu'**au moins à certains moments, l'alimentation (13) en gaz sous pression est reliée par l'intermédiaire d'un écran (28) à l'environnement, **en ce que** l'écran (28) est réalisé séparément de la soupape (17) et **en ce qu'**un clapet anti-retour (26) est disposé dans la zone du raccordement du tuyau respiratoire flexible (5) à la chambre de liaison (16).

2. Dispositif selon la revendication 1, **caractérisé en ce que** l'écran (28) est raccordé en série à une soupape de commutation (29).

3. Dispositif selon les revendications 1 ou 2, **caractérisé en ce qu'**une soupape anti-retour (31) est raccordée en parallèle à l'écran (28) et à la soupape de commutation (29).

4. Dispositif selon l'une des revendications 1 à 3, **caractérisé en ce que** l'écran (28) est raccordé à un ajutage Venturi (15).

5. Dispositif selon l'une des revendications 1 à 4, **caractérisé en ce que** la sonde de pression (22) est disposée dans la zone d'une chambre de liaison (16) qui est disposée entre une source (13) de gaz sous pression et le tuyau respiratoire flexible (5).

6. Dispositif selon l'une des revendications 1 à 5, **caractérisé en ce que** la chambre de liaison (16) est raccordée par un tuyau flexible de commande (27) à la soupape (17) prévue pour le patient.

7. Dispositif selon l'une des revendications 1 à 6, **caractérisé en ce que** tant l'écran (28) que le clapet anti-retour (31) raccordé en parallèle à l'écran (28) sont raccordés à la chambre de liaison (16).

8. Dispositif selon l'une des revendications 1 à 7, **caractérisé en ce que** la soupape anti-retour (31) relie l'ajutage Venturi (15) à l'environnement.

9. Dispositif selon l'une des revendications 4 et 5 à 8 dans la mesure où elles dépendent de la revendication 4, **caractérisé en ce que** l'écran (28) est raccordé à une zone de liaison située entre l'ajutage Venturi (15) et le tuyau respiratoire flexible (5).

10. Dispositif selon l'une des revendications 1 à 9, **caractérisé en ce que** la soupape anti-retour (31) est raccordée en parallèle à l'écran (28).

11. Dispositif selon l'une des revendications 1 à 10, **caractérisé en ce que** l'écran (28) est raccordé en série sur une soupape anti-retour (33).

12. Dispositif selon l'une des revendications 4 et 5 à 11 dans la mesure où elles dépendent de la revendication 4, **caractérisé en ce que** l'écran (28) est raccordé à la chambre de liaison (16) et **en ce que** la soupape anti-retour (31) est raccordée à l'ajutage Venturi (15).

13. Dispositif selon l'une des revendications 1 à 11, **caractérisé en ce que** l'écran (28) et la soupape de commutation (29) sont raccordés à la chambre de liaison (16) et **en ce que** la chambre de liaison (16) est reliée directement à la source (13) de gaz sous pression par une soupape proportionnelle (14).

14. Dispositif selon l'une des revendications 1 à 11, **caractérisé en ce que** la chambre de liaison (16) est raccordée directement à la source (13) de gaz sous pression.

15. Dispositif selon l'une des revendications 1 à 14, **caractérisé en ce qu'**il est réalisé de manière à permettre une assistance respiratoire à domicile.

16. Dispositif selon l'une des revendications 1 à 14, **caractérisé en ce qu'**il est configuré de manière à permettre une assistance respiratoire de secours.

17. Dispositif selon l'une des revendications 1 à 14, **caractérisé en ce qu'**il est configuré de manière à permettre une assistance respiratoire lors d'un transport.

18. Dispositif selon l'une des revendications 1 à 14, **caractérisé en ce qu'**il est configuré de manière à permettre une assistance respiratoire intensive.

19. Procédé de commande d'un appareil respiratoire dans lequel la pression délivrée par une alimentation (13) en gaz sous pression est régulée par recours à au moins un signal détecté par mesure, **caractérisé en ce que** la pression délivrée est commandée par recours à une soupape disposée dans la zone de l'extrémité tournée vers une interface de patient d'un tuyau respiratoire flexible (5), **en ce qu'**au moins à certains moments, une fuite vis-à-vis de l'environnement est formée à travers un conduit d'évacuation d'air comprimé par un écran (28) réalisé séparément de la soupape et **en ce que** le renvoi de gaz respiratoire est bloqué par une soupape anti-retour (26) disposée dans la zone du tuyau respiratoire flexible (5).

20. Procédé selon la revendication 19, **caractérisé en ce que** la pression d'expiration est régulée.

21. Procédé selon les revendications 19 ou 20,
**caractérisé en ce que** la régulation de pression est réalisée par recours à une soupape proportionnelle (19).

22. Procédé selon l'une des revendications 19 à 21, **caractérisé en ce qu'**il réalise la régulation de manière continue.

23. Procédé selon l'une des revendications 19 à 21, **caractérisé en ce qu'**il réalise la régulation de manière quasi continue.
